# EUROPÄISCHE PATENTSCHRIFT

(11) **EP 1 725 512 B1**
(45) Veröffentlichungstag und Bekanntmachung des Hinweises auf die Patenterteilung: **03.12.2014**
(21) Anmeldenummer: 05707705.9
(22) Anmeldetag: 04.03.2005
(51) Int. Cl.: C07C 45/00, C07C 47/21

(54) **VERFAHREN ZUR HERSTELLUNG OPTISCH AKTIVER CARBONYLVERBINDUNGEN**
METHOD FOR PRODUCING OPTICALLY ACTIVE CARBONYL COMPOUNDS
PROCEDE POUR PRODUIRE DES COMPOSES CARBONYLE A ACTIVITE OPTIQUE

(30) Priorität: 08.03.2004 DE 102004011543
(43) Veröffentlichungstag der Anmeldung: 29.11.2006
(73) Patentinhaber: BASF SE, 67056 Ludwigshafen (DE)
(72) Erfinder: JOHANN, Thorsten, 67117 Limburgerhof (DE); LÖBER, Oliver, 55234 Freimersheim (DE); BERGNER, Eike Johannes, 69198 Schriesheim (DE); EBEL, Klaus, 68623 Lampertheim (DE); HARTH, Klaus, Tai Tam, Hong Kong (CN); WALSDORFF, Christian, 67061 Ludwigshafen (DE)
(86) Internationale Anmeldenummer: PCT/EP2005/002288
(87) Internationale Veröffentlichungsnummer: WO 2005/085160

(56) Entgegenhaltungen:
- EP-A- 1 225 163
- WO-A-97/33853
- DE-A1- 4 236 111
- M. J. DOEVRE: "Sur quelques composés des séries citronnellique et rhodinique" BULLETIN DE LA SOCIETE CHIMIQUE DE FRANCE., Bd. 45, 1929, Seiten 1098-1107, XP008048011 FRSOCIETE FRANCAISE DE CHIMIE. PARIS.
- DATABASE CA [Online] CHEMICAL ABSTRACTS SERVICE, COLUMBUS, OHIO, US; OGINO, YOSHISADA ET AL: "Citronellal and citral" XP002330835 gefunden im STN Database accession no. 1977:438878 & JP 51 149217 A2 (KURARAY CO., LTD., JAPAN) 22. Dezember 1976 (1976-12-22)

## Beschreibung

Die vorliegende Erfindung betrifft ein Verfahren zur Herstellung optisch aktiver Aldehyde oder Ketone mit 3 bis 25 Kohlenstoffatomen, die mindestens ein racemisierbares Stereozentrum in α- und/oder β-Position zur Carbonylgrupppe aufweisen.

Chirale Aldehyde oder Ketone stellen wichtige Zwischenprodukte zur Herstellung höher veredelter Verbindungen dar. Daneben besitzen sie ihrerseits in bestimmten Anwendungsgebieten wie z.B. der Riech- bzw. Aromastoffindustrie zentrale Bedeutung als Wert- oder Wirkstoffe.

Aufgrund ihrer schweren Zugänglichkeit werden chirale Aldehyde oder Ketone oft in racemischer Form hergestellt bzw. eingesetzt oder aus natürlichen Quellen in optisch aktiver Form gewonnen. Der Herstellung oder Umsetzung optisch aktiver Aldehyde oder Ketone in technischem Maßstab sind oft enge Grenzen gesetzt, da die genannten Verbindungen chemisch, insbesondere stereochemisch labil und daher mit einer Vielzahl von technisch wie ökonomisch attraktiven Verfahrens- bzw. Reaktionsbedingungen nicht kompatibel sind.

Die DE-A 199 11 169 beschreibt ein Verfahren zur Herstellung cyclischer α,β-ungesättigter Ketone durch Dehydrierung von cyclischen Ketonen bei erhöhter Temperatur in Gegenwart eines Katalysators in der Gasphase.

Die WO 97/33853 betrifft ein Verfahren zur Dehydrierung von sekundären cyclischen Alkoholen in Gegenwart eines Katalysators, enthaltend Zinkoxid und Calciumcarbonat, bei erhöhter Temperatur in der Gasphase.

In der CN-A 1059710 und der CN-A 1059711 wird ein Verfahren zur Herstellung von Campher durch Dehydrierung von Isoborneol in Gegenwart von Katalysatoren auf CaO/ZnO-Basis beschrieben. Die Reaktion wird unter vermindertem Druck von 0 bis 60 Torr durchgeführt.

In Bulletin de la sciete chimique de france, Bd. 45, 1929, Seiten 1098 - 1107 wird ein Verfahren zur Herstellung von d-Citronellal und I-Rhodinal durch katalytische Dehydrierung der entsprechenden Alkohole in Gegenwart von Kupfer als Katalysator beschrieben.

Aufgabe der vorliegenden Erfindung war die Bereitstellung eines Verfahrens, das es ermöglicht, optisch aktive Alkohole mit racemisierbaren Stereozentren durch Dehydrierung und unter weitgehendem Erhalt der absoluten Konfiguration der racemisierbaren Stereozentren in die entsprechenden optisch aktiven Aldehyde oder Ketone zu überführen.

Die Aufgabe wurde in überraschender Weise erfindungsgemäß gelöst durch die Bereitstellung eines Verfahrens zur Herstellung optisch aktiver Aldehyde oder Ketone mit 3 bis 25 Kohlenstoffatomen, die mindestens ein racemisierbares Stereozentrum in α- und/oder β-Position zur Carbonylgrupppe aufweisen, durch katalytische Dehydrierung der entsprechenden optisch aktiven primären oder sekundären Alkohole in Gegenwart eines Katalysators, der Zink und Calcium in oxidischer Form und/oder in Form ihrer Carbonate enthält, in der Gasphase.

Unter optisch aktiven Verbindungen sind im Rahmen der vorliegenden Erfindung solche Verbindungen zu verstehen, die in der Lage sind, als solche oder in gelöster Form die Polarisationsebene des durchtretenden linear polarisierten Lichtes zu drehen. Bei Verbindungen mit einem stereogenen Zentrum handelt es sich dabei um nichtracemische Gemische der beiden Enantiomere, d.h. um Gemische, in denen die beiden Enantiomere zu nicht gleichen Teilen vorliegen. Ein geeignetes Maß zur Beschreibung dieses Verhältnisses ist der sogenannte Enantiomerenüberschuss (ee), dessen Bestimmung durch geeignete Methoden, z.B. auf gaschromatographischem Wege, dem Fachmann an sich bekannt ist.

Unter dem Begriff racemisierbares Stereozentrum ist dabei insbesondere ein asymmetrisch substituiertes Kohlenstoffatom zu verstehen, das unter Einwirkung bestimmter Reagenzien wie z.B. Säuren oder Basen aber auch Radikalen in der Lage ist, zumindest intermediär eine trigonal-planare Konfiguration unter Verlust der ursprünglichen stereochemischen Information einzunehmen. Insbesondere sind hierbei solche asymmetrischen Kohlenstoffatome zu nennen, die neben drei nicht-Wasserstoff-Substituenten ein Wasserstoffatom tragen, das beispielsweise durch geeignete Basen abstrahiert werden kann. Als weiteres Beispiel seien die asymmetrisch substituierten tertiären Carbinolzentren genannt. Nicht racemisierbar im Sinne der vorliegenden Erfindung sind beispielsweise asymmetrische Brückenkopfatome verbrückter bi- bzw. polycyclischer Verbindungen.

Das erfindungsgemäße Verfahren eignet sich zur Dehydrierung von optisch aktiven primären und sekundären Alkoholen mit 3 bis 25 Kohlenstoffatomen, die mindestens ein racemisierbares Stereozentrum in α- und/oder β-Position zu der zu dehydrierenden Alkoholfunktion tragen. Im Falle der Umsetzung sekundärer Alkohole ist damit nicht das gegebenenfalls ebenfalls asymmetrisch substituierte Kohlenstoffatom gemeint, das die zu dehydrierendende Alkoholfunktion trägt. Setzt man chirale Alkohole in racemischer Form ein so erhält man mit gutem Erfolg die entsprechenden racemischen Aldehyde und Ketone. Bevorzugt setzt man die chiralen primären oder sekundären Alkohole in optisch aktiver Form ein und erhält unter weitgehendem Erhalt der Konfiguiration des racemisierbaren Stereozentrums, d.h. unter weitgehender Unterdrückung der Racemisierung des besagten Stereozentrums, die entsprechenden optisch aktiven Aldehyde oder Ketone.

Aus den primären oder sekundären Alkoholen in optisch aktiver Form, die ein racemisierbares Stereozentrum in α- oder β-Position zu der zu dehydrierenden Alkoholfunktion tragen, sind durch das erfindungsgemäße Verfahren solche Aldehyde oder Ketone in optisch aktiver Form zugänglich, die ein Stereozentrum in α- bzw. β-Position zu der resultierenden Carbonylfunktion aufweisen. Das weitere Substitutionsmuster bzw. die Anzahl der Substituenten der gewählten Substrate ist nicht kritisch und in der Regel nur durch die Stabilität der Substituenten bzw. der umzusetzenden Verbindung unter den gewählten Reaktionsbedingungen begrenzt.

Das erfindungsgemäße Verfahren eignet sich insbesondere zur Dehydrierung jeweils optisch aktiver cycloaliphatischer sowie offenkettiger primärer und sekundärer Alkohole mit mindestens einem racemisierbaren Stereozentrum in α- und/oder β-Position zu der zu dehydrierenden Alkoholfunktion, die jeweils verzweigt oder unverzweigt sein können und 3 bis 25, bevorzugt 5 bis 12 Kohlenstoffatome tragen können zu den entsprechenden optisch Aldehyden oder Ketonen. Man erhält dann in erfindungsgemäßer Weise verzweigt oder unverzweigt offenkettige oder monocyclische Aldehyde oder Ketone mit mindestens einem racemisierbaren Stereozentrum in α- und/oder β-Position zur Carbonylgrupppe. Die zu dehydrierenden Alkohole können auch eine oder mehrere, in der Regel 1 bis 3 olefinische Doppelbindungen aufweisen, die unter den Reaktionsbedingungen stabil sind.

Die genannten Verbindungen können als Einzelverbindungen oder in Form von Gemischen derselben, insbesondere, im Fall der Umsetzung von Verbindungen mit mehr als einem Stereozentrum, in Form von Diastereomerengemischen in erfindungsgemäßer Weise eingesetzt werden.

Bevorzugte Ausgangsverbindungen zur Durchführung des erfindungsgemäßen Verfahrens sind optisch aktive Terpenalkohole, insbesondere optisch aktive Mono-, oder Sesquiterpenalkohole, d.h. Alkohole mit 5, 10 oder 15 Kohlenstoffatomen sowie deren Derivate. Beispielhaft seien hierfür die folgenden Verbindungen genannt: 2-Methyl-butan-1-ol, 3,7-Dimethyl-oct-6-en-1-ol (Citronellol), 3,7-Dimethyl-octan-1-ol, 8-p-Menthen-3-ol (Isopulegol), p-Menthan-3-ol (Menthol), 2-Methylcyclohexanol, 3-Methylcyclohexanol, 2-Methyl-cyclopentanol, 3-Methylcyclopentanol, 2,6-Dimethylcyclohexanol oder 2,3-Dimethylcyclohexanol.

Aus den genannten Verbindungen werden in erfindungsgemäßer Weise durch Dehydrierung die entsprechenden Aldehyde bzw. Ketone erhalten, die im folgenden ebenfalls beispielhaft genannt seien: 2-Methyl-butan-1-al, 3,7-Dimethyl-oct-6-en-1-al (Citronellal), 3,7-Dimethyl-octan-1-al, 8-p-Menthen-3-on (Isopulegon), p-Menthan-3-on (Menthon), 2-Methylcyclohexanon, 3-Methylcyclohexanon, 2-Methyl-cyclopentanon, 3-Methylcyclopentanon, 2,6-Dimethylcyclohexanon oder 2,3-Dimethylcyclohexanon.

In besonderem Maße eignet sich das Verfahren zur Herstellung von optisch aktivem Citronellal der Formel (I) 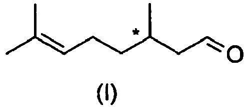 ausgehend von optisch aktivem Citronellol der Formel (II).

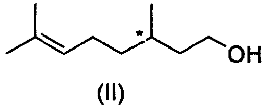

Dabei sind naturgemäß beide Enantiomere des Citronellols gleichermaßen als Ausgangsstoffe im Rahmen des erfindungsgemäßen Verfahrens geeignet. Ein bevorzugter Ausgangsstoff ist R-(+)- Citronellol (D-Citronellol).

Bevorzugt setzt man im Rahmen der vorliegenden Erfindung solche Alkohole ein, die einen Enantiomerenüberschuss von mindestens 85 % ee, besonders bevorzugt von mindestens 90 % ee und ganz besonders bevorzugt von mindestens 95 % ee aufweisen. Durch die Wahl des Enantiomerenüberschusses im Edukt lässt sich der Enantiomerenüberschuss der erfindungsgemäß erhaltenen Produkt-Aldehyde bzw. Ketone beeinflussen. Mit besonderem Vorteil wählt man die Reaktionsbedingungen, beispielsweise die Reaktionstemperatur, so, dass der Enantiomerenüberschuss des erhaltenen Produkt-Aldehyds oder -Ketons mindestens etwa 90 %, bevorzugt mindestens etwa 95 % des Enantiomerenüberschusses des eingesetzten Alkohols beträgt.

Zur Durchführung des erfindungsgemäßen Verfahrens eignen sich Katalysatoren die Zink und Calcium in oxidischer Form und/oder in Form ihrer Carbonate enthalten. Neben den genannten Elementen können die erfindungsgemäß einsetzbaren Katalysatoren noch eines oder mehrere Elemente der Gruppen 1, 2, 3, 4, 13 und/oder 14, wie z.B. Na, K, Mg, Ti, Zr, Al, C, Si, und/oder Ge enthalten. . Dabei sind insbesondere solche Katalysatoren zu bevorzugen, die Zinkoxid und Calciumcarbonat enthalten.

Bevorzugte Katalysatoren zur Durchführung des erfindungsgemäßen Verfahrens sind solche, deren aktive Komponente zu 30 bis 60 Gew.-%, bevorzugt zu 40 bis 50 Gew.-% aus Zinkoxid und zu 40 bis 70 Gew.-%, bevorzugt zu 50 bis 60 Gew.-% aus Calciumcarbonaten bestehen. Weiterhin bevorzug sind darunter solche, deren Calciumcarbonat-Komponente in der Calcit-Modifikation vorliegt. Die genannten Mengenanteile sind aus der geglühten Katalysatormasse zu bestimmen, in der Zink und Calcium jeweils in Form ihrer Oxide vorliegen.

Geeignete Trägermaterialien der erfindungsgemäßen Katalysatoren sind beispielsweise in DE A 197 57 297 beschrieben. Als weiteres Trägermaterial kommen beispielsweise auch Calciumcarbonat sowie weitere geeignete Trägermaterialien in Betracht.

In einer bevorzugten Ausführungsform weisen die erfindungsgemäß einsetzbaren Katalysatoren eine spezifische Oberfläche nach BET von 5 bis 50 m²/g, vorzugsweise von 10 bis 30 m²/g auf.

Ein solcher Katalysator ist beispielsweise durch Ausfällen von schwerlöslichen Zink- und Calciumverbindungen aus wasserlöslichen Zink- und Calciumverbindungen mit einer Base und anschließender Aufarbeitung in an sich bekannter Weise erhältlich, wobei man
(a) als Base ein wasserlösliches basisches Carbonat einsetzt,
(b) gewünschtenfalls die schwerlöslichen Zink- und Calciumverbindungen nach dem Ausfällen filtriert,
(c) die gewünschtenfalls filtrierten Zink- und Calciumverbindungen wäscht,
(d) die gewaschenen Zink- und Calciumverbindungen aus (c) trocknet unter Erhalt eines Pulvers, und anschließend
(e) das Pulver aus (d) bei Temperaturen von nicht über 600°C calciniert, und
(f) gewünschtenfalls das calcinierte Pulver zu Formkörpern verpresst.

Als wasserlösliche Zink- und Calciumsalze kann man Acetate, Sulfate, Nitrate, Chloride, bevorzugt Nitrate wie Zinknitrat, Zinkacetat, Zinksulfat, Calciumacetat, Calciumnitrat, bevorzugt Zinknitrat und Calciumnitrat, einsetzen. Üblicherweise kann man wässrige Lösungen der entsprechenden Salze in Konzentrationen im Bereich von 3 bis 25, bevorzugt von 10 bis 25, insbesondere 20 Gew.-%, einsetzen.

Das Molverhältnis von Zink zu Calcium wählt man bevorzugt so, dass nach dem Calcinieren die aktive Komponente des Katalysators zu 30 bis 60 Gew.-% aus Zinkoxid und zu 40 bis 70 Gew.-% aus Calciumcarbonat, welches bevorzugt in der Calcit-Modifikation vorliegt, besteht.

Als Base verwendet man vorzugsweise wasserlösliche basische Carbonate wie Alkalimetallcarbonate wie Natriumcarbonat, Kaliumcarbonat, Alkalimetallhydrogencarbonate wie Natriumhydrogencarbonat, Kaliumhydrogencarbonat, Ammoniumcarbonat oder Ammoniumhydrogencarbonat sowie deren Mischungen, vorzugsweise Natriumcarbonat, besonders bevorzugt in Form ihrer wässrigen Lösungen in Konzentrationen im allgemeinen im Bereich von 0,5 bis 30, bevorzugt von 10 bis 25 Gramm Base/100 Gramm Lösung.

Die Fällung führt man im allgemeinen bei Temperaturen im Bereich von 10 bis 90°C, vorzugsweise von 40 bis 80°C durch. Nach dem Ausfällen kann man gewünschtenfalls den Niederschlag abfiltrieren. Der gewünschtenfalls abfiltrierte Niederschlag wäscht man in der Regel mit Wasser, bevorzugt solange, bis kein Nitrat mehr mittels der Nitratringprobe feststellbar ist, und trocknet ihn anschließend bevorzugt bei einer Temperatur im Bereich von 90 bis 150°C unter Erhalt eines getrockneten Pulvers. Die Trocknung kann in ruhender oder bewegter Schicht, vorzugsweise durch Sprühtrocknung, erfolgen.

Das getrocknete Pulver kann dann bei Temperaturen von nicht höher als 600°C, bevorzugt im Bereich von 300 bis 600°C, insbesondere von 400 bis 475°C, bevorzugt in Luft, calciniert werden. Nach bisherigen Beobachtungen führt eine längere Erhitzung über 600°C zur Bildung der Aragonit-Modifikation von CaCO₃. Eine kurzfristige Erhitzung über 600°C ist dann nicht hinderlich für die Herstellung der bevorzugten Katalysatoren, solange sich dabei kein Aragonit (Nachweis mittels Röntgendiffraktometrie) bildet.

Nach dem Calcinieren kann man gewünschtenfalls das calcinierte Pulver zu Formkörpern wie Tabletten, Ringe, Zylinder etc., bevorzugt Tabletten, verpressen.

In einer bevorzugten Ausführungsform verpresst man das calcinierte Pulver zusammen mit Graphit, bevorzugt mit 0,1 bis 5, besonders bevorzugt mit 1 bis 2,5, insbesondere 2 Gew.-%, bezogen auf die Gesamtmasse, Graphit.

In einer weiteren bevorzugten Ausführungsform verpresst man das uncalcinierte Pulver aus Schritt (c) (s.o.) zu Formkörpern, bevorzugt zu Tabletten, Ringtabletten, kalottierten Tabletten, wie in der US-6,518,220 beschrieben, oder Triloben und calciniert die so erhaltenen Formkörper wie unter Schritt (d) beschrieben. Alternativ kann auch eine Extrusion zu Strängen oder Sternsträngen, bevorzugt zu Strängen, durchgeführt werden.

Die so erhaltenen calcinierten Pulver und Formkörper können als Katalysatoren eingesetzt werden, wobei diese Katalysatoren als aktive Komponenten Zinkoxid und Calciumcarbonat (in der Calcit-Modifikation) und als passive Komponente gewünschtenfalls Graphit enthalten können.

In einer weiteren bevorzugten Ausführungsform setzt man einen Katalysator der beschriebenen Art ein, der ein Porenvolumen im Bereich von 0,10 bis 0,50 cm³/g, insbesondere von 0,20 bis 0,35 cm³/g, bei einem Porendurchmesser im Bereich von 5 nm bis 300 mm aufweist, wobei besonders bevorzugt mindestens 85 %, vorzugsweise mehr als 90 % dieses Porenvolumens mit einem Porendurchmesser im Bereich von 0,01 bis 0,5 mm verbunden ist.

Besonders bevorzugte Katalysatoren der genannten Art sind solche, die eine Stirndruckfestigkeit im Bereich von 500 bis 4000 N/cm², insbesondere von 1000 bis 2500 N/cm² und eine Seitendruckfestigkeit von 30 bis 300 N, vorzugsweise 50 bis 200 N aufweisen.

Die spezifische Oberfläche nach BET beträgt im allgemeinen 5 bis 50 m²/g, vorzugsweise 10 bis 30 m²/g. Das Porenvolumen im Porendurchmesserbereich zwischen 0,1 nm und 300 nm besitzt Werte üblicherweise zwischen 0,1 und 0,5 cm³/g, vorzugsweise 0,2 bis 0,35 cm³/g mit der Maßgabe, dass mindestens 85 %, vorzugsweise mehr als 90 % dieses Porenvolumens im Porendurchmesserbereich von 0,01 bis 0,5 mm liegen.

Die Stirndruckfestigkeit der Tabletten beträgt vorzugsweise 500 bis 4000 N/cm², insbesondere 1000 bis 2500 N/cm² und die Seitendruckfestigkeit der Pillen liegt bevorzugt zwischen 30 und 300 N, vorzugsweise 50 bis 200 N.

Mit besonderem Vorteil wäscht man den Niederschlag aus schwerlöslichen Zink- und Calciumverbindungen, bevorzugt Zinkhydroxidcarbonat und Calciumcarbonat, auf Filterpressen, maischt den dabei erhaltenen Filterkuchen mit Wasser an, und versprüht die Maische zum Trocknen in einem Sprühturm. Das auf diese Weise erhaltene getrocknete Sprühpulver kann man danach wie oben beschrieben weiterverarbeiten.

Erfindungsgemäß bringt man den gasförmigen primären oder sekundären Alkohol in an sich üblicher Weise in Kontakt mit dem eingesetzten Katalysator, beispielsweise in einem Festbettreaktor, Rohrreaktor, Rohrbündelreaktor oder in einem Wirbelschichtreaktor, bevorzugt in einem Rohrreaktor, in dem der Katalysator fest angeordnet ist. Besonders bevorzugt sind Rohrbündelreaktoren. Der Austrag wird üblicherweise destillativ aufgearbeitet.

Im allgemeinen verdampft man den erfindungsgemäß einzusetzenden optisch aktiven Alkohol in an sich bekannter Weise, beispielsweise in einem geeigneten Verdampfer.

Das erfindungsgemäße Verfahren führt man üblicherweise bei erhöhter Temperatur durch. Die Temperatur der Gasphase in der Reaktionszone wählt man üblicherweise im Bereich von 250 bis 600°C, vorzugsweise von 300 bis 450°C. In einer bevorzugten Ausführungsform wählt man den Temperaturbereich so, dass man einen Umsatz im Bereich von 20 bis 60, bevorzugt von 35 bis 50 %, Alkohol erhält. Im Falle von Citronellol als Ausgangsverbindung wählt man dann die Temperatur vorzugsweise im Bereich von 350 bis 450°C.

Den Druck der Gasphase in der Reaktionszone wählt man im allgemeinen im Bereich von 0,3 bis 10 bar.

Die Belastung des Katalysators wählt man im allgemeinen im Bereich von 0,5 bis 3,0, vorzugsweise von 0,6 bis 2,0 Liter Alkohol pro Liter an Katalysator und pro Stunde. Geeignete Reaktorformen zur Durchführung des erfindungsgemäßen Verfahrens sind der Festbettrohr- oder Rohrbündelreaktor. Bei diesen befindet sich der gewählte Katalysator als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird oder ein Wärmeträgermedium (Salzbad, Wälzgas etc.) eingesetzt wird. Es kann auch eine elektrische Beheizung der Reaktionsrohre mit Heizmanschetten erfolgen. Übliche Reaktionsrohr-Innendurchmesser betragen etwa 2,5 bis 15 cm. Ein typischer Dehydrierrohrbündelreaktor umfasst ca. 10 bis 32000 Reaktionsrohre, bevorzugt ca. 10 bis 200 Reaktionsrohre. Die Temperatur im Reaktionsrohrinneren bewegt sich üblicherweise im Bereich von 250 bis 600°C, vorzugsweise im Bereich von 300 bis 600°C. Der Arbeitsdruck liegt üblicherweise zwischen 0,5 und 8 bar, häufig zwischen 1 und 2 bar.

Das erfindungsgemäße Verfahren kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 250 bis 600°C.

Die erfindungsgemäße katalytische Dehydrierung kann mit oder ohne sauerstoffhaltigem Gas als Co-Feed und optional unter Zusatz von Wasserdampf, Stickstoff, Methan und/oder Argon durchgeführt werden. Der gewählte Reaktor kann ein oder mehrere aufeinanderfolgende Katalysatorbetten aufweisen. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde.

In einer besonders bevorzugten Ausführungsform des erfindungsgemäßen Verfahrens setzt man einen optisch aktiven Terpenalkohol, insbesondere Citronellol, bevorzugt R-(+)-Citronellol mit einem Enantiomerenüberschuss von mindestens 90 % ee, bevorzugt 95 % ee an einem Katalysator, dessen Aktivkomponenete 54 bis 57 Gew.-% Zinkoxid und 43 bis 46 Gew.-% Calciumcarbonat enthält (jeweils bestimmt in Form der Oxide der geglühten Katalysatormasse) in einem geeigneten Reaktor, beispielsweise einem Rohrreaktor um. Der Reaktor kann dabei durch jede geeignete Methode, bevorzugt durch eine Salzschmelze, auf Temperaturen im Bereich von etwa 350 bis etwa 450°C beheizt werden. Die Reaktion findet in der Gasphase statt. Gute Resultate erhält man insbesondere, wenn die Reaktion in Abwesenheit von Sauerstoff durchgeführt wird. Dazu leitet man ein das zu dehydrierende Edukt enthaltende Stoffgemisch beispielsweise in einem Inertgasstrom wie z.B. einem Stickstoffstrom über den gewählten Katalysator. Optional ist auch eine autotherme Fahrweise durch partielle H₂-Verbrennung nach vorheriger Einspeisung eines H₂-haltigen Stoffgemisches möglich.

Die Isolierung der Reaktionsprodukte kann nach allen geeigneten und dem Fachmann an sich bekannten Methoden vorgenommen werden. Man erhält auf diese Weise bei einem Umsatz von vorzugsweise etwa 30 bis etwa 60 % d. Th. optisch aktives Citronallal in einer Selektivität von normalerweise etwa 60 bis etwa 95 %.

Die in erfindungsgemäßer Weise herstellbaren optisch aktiven Aldehyde oder Ketone lassen sich in vielfältiger Weise verwerten. Sie stellen mitunter wichtige Ausgangs- bzw. Zwischenprodukte zur Synthese höher veredelter Produkte dar. So stellt beispielsweise optisch aktives Citronellal, bevorzugt R-(+)-Citronellal ein wichtiges Intermediat zur Synthese von optisch aktivem Menthol, bevorzugt L-Menthol dar. So lässt sich optisch aktives Citronellal unter Einsatz geeigneter, üblicherweise saurer, bzw. lewis-saurer Katalysatoren zu optisch aktivem Isopulegol cyclisieren. Daraus ist durch Hydrierung optisch aktives Menthol zugänglich.

Das folgende Beispiel dient der Verdeutlichung der Erfindung, ohne sie jedoch in irgendeiner Weise zu beschränken:

### Beispiel 1:

Ein durch eine Salzschmelze beheizbarer Rohrreaktor wurde mit 10,8 g eines Katalysators bestehend aus 55 Gew.-% ZnO, und 45 Gew.-% CaCO₃ in der Calcit-Modifikation (jeweils bestimmt in Form der Oxide der geglühten Katalysatormasse) beschickt. Bei einer Temperatur von 400°C wurden ein Gemisch von 46 Nl/h Stickstoff und 3,44 g/h *R*-Citronellol mit einem Enantiomerenüberschuss von 95% ee über die Schüttung geleitet. Man erhielt bei einem Umsatz von 50,2 % in einer Selektivität von 75,5 % Citronellal mit einem Enantiomerenüberschuss bezüglich R-Citronellal von 95 % ee.

## Patentansprüche

1. Verfahren zur Herstellung optisch aktiver Aldehyde oder Ketone mit 3 bis 25 Kohlenstoffatomen, die mindestens ein racemisierbares Stereozentrum in α- und/oder β-Position zur Carbonylgruppe aufweisen, durch katalytische Dehydrierung der entsprechenden optisch aktiven primären oder sekundären Alkohole in Gegenwart eines Katalysators, der Zink und Calcium in oxidischer Form und/oder in Form ihrer Carbonate enthält, in der Gasphase.

2. Verfahren nach Anspruch 1, **dadurch gekennzeichnet, dass** man einen Katalysator einsetzt, der Zinkoxid und Calciumcarbonat enthält.

3. Verfahren nach Anspruch 1 bis 2, **dadurch gekennzeichnet, dass** man einen Katalysator einsetzt, dessen aktive Komponente zu 30 bis 60 Gew.-% aus Zinkoxid und zu 40 bis 70 Gew.-% aus Calciumcarbonaten besteht.

4. Verfahren nach Anspruch 2 und 3, **dadurch gekennzeichnet, dass** das Calciumcarbonat in der Calcit-Modifikation vorliegt.

5. Verfahren nach Anspruch 1 bis 4 zur Herstellung verzweigt oder unverzweigt offenkettiger oder monocyclischer Aldehyde oder Ketone.

6. Verfahren nach Anspruch 1 bis 5 zur Herstellung von optisch aktivem 2-Methylbutan-1-al, 3,7-Dimethyl-oct-6-en-1-al, 3,7-Dimethyl-octan-1-al, 8-p-Menthen-3-on, p-Menthan-3-on, 2-Methylcyclohexanon, 3-Methylcyclohexanon, 2-Methyl-cyclopentanon, 3-Methylcyclopentanon, 2,6-Dimethylcyclohexanon oder 2,3-Dimethylcyclohexanon.

7. Verfahren nach Anspruch 1 bis 6 zur Herstellung von optisch aktivem Citronellal ausgehend von optisch aktivem Citronellol.

8. Verfahren nach Anspruch 1 bis 7, **dadurch gekennzeichnet, dass** der Enantiomerenüberschuss (ee) des erhaltenen des erhaltenen Aldehyds oder Ketons mindestens 90% des Enantiomerenüberschusses des eingesetzten Alkohols entspricht.

9. Verfahren nach Anspruch 1 bis 8, **dadurch gekennzeichnet, dass** man die Dehydrierung bei einer Temperatur im Bereich von 250 bis 600°C durchführt.

10. Verfahren zur Herstellung von optisch aktivem Menthol welches umfasst die Herstellung von Citronellal nach Anspruch 1 bis 9, die Cyclisierung zum Isopulegol und anschließende Hydrierung.

## Claims

1. A process for preparing optically active aldehydes or ketones which have from 3 to 25 carbon atoms and at least one racemizable stereocenter in the α and/or β position relative to the carbonyl group by catalytic dehydrogenation of the corresponding optically active primary or secondary alcohols in the gas phase in the presence of a catalyst comprising zinc and calcium in oxidic form and/or in the form of their carbonates.

2. The process according to claim 1, wherein a catalyst comprising zinc oxide and calcium carbonate is used.

3. The process according to claim 1 or 2, wherein a catalyst whose active component comprises from 30 to 60% by weight of zinc oxide and from 40 to 70% by weight of calcium carbonates is used.

4. The process according to claim 2 or 3, wherein the calcium carbonate is present in the calcite modification.

5. The process according to any of claims 1 to 4 for preparing branched or unbranched open-chain or monocyclic aldehydes or ketones.

6. The process according to any of claims 1 to 5 for preparing optically active 2-methylbutan-1-al, 3,7-dimethyloct-6-en-1-al, 3,7-dimethyloctan-1-al, 8-p-menthen-3-one, p-menthan-3-one, 2-methylcyclohexanone, 3-methylcyclohexanone, 2-methylcyclopentanone, 3-methylcyclopentanone, 2,6-dimethylcyclohexanone or 2,3-dimethylcyclohexanone.

7. The process according to any of claims 1 to 6 for preparing optically active citronellal from optically active citronellol.

8. The process according to any of claims 1 to 7, wherein the enantiomeric excess (ee) of the aldehyde or ketone obtained corresponds to at least 90% of the enantiomeric excess of the alcohol used.

9. The process according to any of claims 1 to 8, wherein the dehydrogenation is carried out at a temperature in the range from 250 to 600°C.

10. A process for preparing optically active menthol, which comprises the preparation of citronellal according to any of claims 1 to 9, cyclization to form isopulegol and subsequent hydrogenation.

## Revendications

1. Procédé pour la préparation d'aldéhydes ou de cétones optiquement actifs comprenant 3 à 25 atomes de carbone, qui présentent au moins un stéréocentre pouvant être racémisé en position α et/ou en position ß par rapport au groupe carbonyle, par déshydrogénation catalytique des alcools primaires ou secondaires optiquement actifs correspondants en présence d'un catalyseur qui contient du zinc et du calcium sous forme d'oxydes et/ou sous forme de leurs carbonates, en phase gazeuse.

2. Procédé selon la revendication 1, **caractérisé en ce qu'**on utilise un catalyseur qui contient de l'oxyde de zinc et du carbonate de calcium.

3. Procédé selon la revendication 1 à 2, **caractérisé en ce qu'**on utilise un catalyseur dont le composant actif est constitué à raison de 30 à 60% en poids d'oxyde de zinc et à raison de 40 à 70% en poids de carbonate de calcium.

4. Procédé selon la revendication 2 et 3, **caractérisé en ce que** le carbonate de calcium se trouve dans la modification calcite.

5. Procédé selon la revendication 1 à 4 pour la préparation d'aldéhydes ou de cétones, ramifiés ou non ramifiés, à chaîne ouverte ou monocycliques.

6. Procédé selon la revendication 1 à 5 pour la préparation de 2-méthylbutan-1-al, de 3,7-diméthyloct-6-én-1-al, de 3,7-diméthyloctan-1-al, de 8-p-menthén-3-one, de p-menthan-3-one, de 2-méthylcyclohexanone, de 3-méthylcyclohexanone, de 2-méthylcyclopentanone, de 3-méthylcyclopentanone, de 2,6-diméthylcyclohexanone ou de 2,3-diméthylcyclohexanone optiquement actifs.

7. Procédé selon la revendication 1 à 6 pour la préparation de citronellal optiquement actif partant de citronellol optiquement actif.

8. Procédé selon la revendication 1 à 7, **caractérisé en ce que** l'excès d'énantiomères (ee) de l'aldéhyde ou de la cétone obtenu(e) correspond à au moins 90% de l'excès d'énantiomères de l'alcool utilisé.

9. Procédé selon la revendication 1 à 8, **caractérisé en ce qu'**on réalise la déshydrogénation à une température dans la plage de 250 à 600°C.

10. Procédé pour la préparation de menthol optiquement actif, qui comprend la préparation de citronellal selon la revendication 1 à 9, la cyclisation en isopulégol et l'hydrogénation consécutive.
